(19) **Europäisches Patentamt / European Patent Office / Office européen des brevets**

(11) **EP 4 428 248 A1**

(12) **EUROPEAN PATENT APPLICATION**
published in accordance with Art. 153(4) EPC

(43) Date of publication:
**11.09.2024 Bulletin 2024/37**

(21) Application number: **22886469.0**

(22) Date of filing: **08.09.2022**

(51) International Patent Classification (IPC):
**C12Q 1/6851** (2018.01)      **C12N 15/113** (2010.01)
**C12Q 1/6886** (2018.01)

(52) Cooperative Patent Classification (CPC):
**C12N 15/113; C12Q 1/6851; C12Q 1/6886**

(86) International application number:
**PCT/JP2022/033651**

(87) International publication number:
**WO 2023/074135 (04.05.2023 Gazette 2023/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.11.2021 JP 2021178839**

(71) Applicants:
• **National Cancer Center**
  **Tokyo 104-0045 (JP)**

• **SYSMEX CORPORATION**
  **Kobe-shi**
  **Hyogo 651-0073 (JP)**

(72) Inventor: **TSUCHIYA, Naoto**
  **Tokyo 104-0045 (JP)**

(74) Representative: **Hoffmann Eitle**
  **Patent- und Rechtsanwälte PartmbB**
  **Arabellastraße 30**
  **81925 München (DE)**

Remarks:
The complete document including Reference Table(s) and the Sequence Listing(s) can be downloaded from the EPO website

(54) **METHOD AND REAGENT KIT FOR ASSISTING DETERMINATION OF LUNG CANCER PROGNOSIS**

(57)    The present invention relates to a method for assisting determination of prognosis of lung cancer, the method including measuring miR-21C in a measurement sample prepared from a specimen of a lung cancer patient, an expression level of miR-21C acquired by the measurement serving as an index of the prognosis of lung cancer. The present invention also relates to a reagent kit used in this method.

Fig. 3B

EP 4 428 248 A1

**Description**

TECHNICAL FIELD

The present invention relates to a method for assisting determination of prognosis of lung cancer. The present invention relates to a reagent kit used for the method.

BACKGROUND ART

Lung cancer is a malignant tumor that occurs in bronchi and alveoli. Among various cancers, lung cancer is known to result in high mortality, and is more likely to cause postoperative recurrence. Adjuvant chemotherapy has therefore been applied to lung cancer patients, in order to prevent the recurrence, and to improve the prognosis. Applicability criteria have, however, remained unclear. The adjuvant chemotherapy, although useful for the lung cancer patients at high recurrence risk, is considered to be excessive for the patients at low recurrence risk. It is therefore necessary to reasonably assess the prognosis of the lung cancer patients.

In recent years, microRNA (also denoted as miRNA or miR) has attracted attention as a prognostic factor of cancer. The microRNA is a non-coding RNA having a short chain length of approximately 20 to 25 bases. For example, Patent Document 1 describes that miR-21 is highly expressed in lung cancer tissue, than in non-cancer tissues. Patent Document 1 also describes that a plurality of miRNAs including miR-21 correlate with survival rate of lung adenocarcinoma patients, and that prognosis of the adenocarcinoma patients with high expression of any of these miRNAs is significantly poor.

PRIOR ART DOCUMENT

PATENT DOCUMENT

Patent Document 1: U.S. Patent No. 8,361,710

SUMMARY OF THE INVENTION

PROBLEMS TO BE SOLVED BY THE INVENTION

Currently, there are still few biomarkers relevant to the prognosis of lung cancer. Hence, a novel biomarker for determining the prognosis of lung cancer has been keenly awaited to be developed.

SOLUTION TO THE PROBLEMS

The present inventors found that the higher the expression level of miR-21C, which is a variant of miR-21, in cancer tissues of the lung cancer patients, the poorer the prognosis. The present inventors also found that the prognosis of lung cancer is predictable with reference to the expression level of miR-21C, and arrived at the present invention.

The present invention provides a method for assisting determination of prognosis of lung cancer, the method including measuring miR-21C in a measurement sample prepared from a specimen of a lung cancer patient, in which an expression level of miR-21C acquired by the measurement serves as an index of the prognosis of lung cancer.

The present invention provides a method for assisting determination of prognosis of lung cancer, the method including the steps of: measuring miR-21C in a measurement sample prepared from a specimen of a lung cancer patient; and determining prognosis of lung cancer, with reference to an expression level of miR-21C acquired by the measurement.

The present invention provides a reagent kit for use in the method according to any of the aforementioned methods, the reagent kit including a primer set that specifically hybridizes to a cDNA derived from miR-21C.

EFFECTS OF THE INVENTION

The present invention enables determination of the prognosis of lung cancer.

BRIEF DESCRIPTION OF THE DRAWINGS

Fig. 1A is a schematic drawing illustrating an exemplary reagent kit of this embodiment.

Fig. 1B is a schematic drawing illustrating an exemplary reagent kit of this embodiment.

Fig. 2A shows survival curves illustrating overall survival rates (OSs) of a group of lung cancer patients with high miR-21C measurement values, and a group of lung cancer patients with low miR-21C measurement values.

Fig. 2B shows survival curves illustrating OSs of a group of early-stage lung cancer patients with high miR-21C measurement values, and a group of early-stage lung cancer patients with low miR-21C measurement values.

Fig. 2C shows survival curves illustrating OSs of a group of lung cancer patients with high miR-21 measurement values, and a group of lung cancer patients with low miR-21 measurement values.

Fig. 2D shows survival curves illustrating OSs of a group of early-stage lung cancer patients with high miR-21 measurement values, and a group of early-stage lung cancer patients with low miR-21 measurement values.

Fig. 3A shows survival curves illustrating recurrence-free survival rates (RFSs) of a group of lung cancer patients with high miR-21C measurement values, and a group of lung cancer patients with low miR-21C measurement values.

Fig. 3B shows survival curves illustrating RFSs of a group of early-stage lung cancer patients with high miR-21C measurement values, and a group of early-stage lung cancer patients with low miR-21C measurement values.

Fig. 3C shows survival curves illustrating RFSs of a group of lung cancer patients with high miR-21 measurement values, and a group of lung cancer patients with low miR-21 measurement values.

Fig. 3D shows survival curves illustrating RFSs of a group of early-stage lung cancer patients with high miR-21 measurement values, and a group of early-stage lung cancer patients with low miR-21 measurement values.

Fig. 4A shows survival curves illustrating RFSs of a group of lung cancer patients with high relative values of miR-21C measurement values to miR-21 measurement values, and a group of lung cancer patients with low relative values.

Fig. 4B shows survival curves illustrating RFSs of a group of early-stage lung cancer patients with high relative values, and a group of early-stage lung cancer patients with low relative values.

Fig. 5A shows survival curves illustrating RFSs of a group of lung cancer patients with high dominant scores (hereinafter, also referred to as "D-scores"), and a group of lung cancer patients with low D-scores.

Fig. 5B shows survival curves illustrating RFSs of a group of early-stage lung cancer patients with high D-scores, and a group of early-stage lung cancer patients with low D-scores.

Fig. 5C shows survival curves illustrating RFSs of a group of well-differentiated lung cancer patients with high D-scores, and a group of well-differentiated lung cancer patients with low D-scores.

Fig. 6A shows survival curves illustrating RFSs of a group of early-stage lung cancer patients with high relative values, and a group of early-stage lung cancer patients with low relative values.

Fig. 6B shows survival curves illustrating RFSs of a group of early-stage lung cancer patients with p53 gene mutation, and a group of early-stage lung cancer patients without p53 gene mutation.

MODE FOR CARRYING OUT THE INVENTION

In this specification, the "prognosis of lung cancer" conceptionally covers survival rate of patients, risk of recurrence of lung cancer, and period up to recurrence or death, after initial treatment of lung cancer. The "initial treatment" refers to treatment given for the first time after diagnosed with lung cancer. The "prognosis of lung cancer" for lung cancer in an inoperable stage conceptionally covers, for example, survival rate after diagnosis, and period from diagnosis to death. The "recurrence of lung cancer" conceptionally covers metastasis of lung cancer. In this embodiment, "poor prognosis of lung cancer" means that the patient is less likely to be alive, or likely to have a recurrence of lung cancer, after the elapse of a predetermined period after the initial treatment of lung cancer. "Good prognosis of lung cancer" means that the patient is likely to be alive, or less likely to have a recurrence of lung cancer, after the elapse of a predetermined period after the initial treatment of lung cancer. The predetermined period may be, but not particularly limited to, one year, two years, three years, four years, five years, six years, seven years, eight years, nine years, or ten years for example.

The method for assisting determination of prognosis of lung cancer of this embodiment (hereinafter, also simply referred to as "the method of this embodiment") measures miR-21C in a measurement sample prepared from a specimen of a lung cancer patient.

Type of lung cancer may be, for example, any of adenocarcinoma, squamous cell carcinoma, large cell carcinoma or small cell carcinoma, without special limitation. Among them, the method is more suitable for determination of prognosis of patients with adenocarcinoma. The lung cancer may have a level of differentiation without special limitation, which may be any of well-differentiated cancer, moderately-differentiated cancer, or poorly-differentiated cancer. The lung cancer may have a stage without special limitation, which may for example any of stage-I (stage-Ia or stage-Ib), stage-II (stage-IIa or stage-IIb), stage-III (stage-IIIa or stage-IIIb), or stage-IV Meanwhile, the prognosis of lung cancer has been known to become poorer as the stage progresses. The method of this embodiment is, therefore, considered to be more useful for patients with early-stage lung cancer, exemplified by those in stage-I or stage-II lung cancer.

The lung cancer patient may be an untreated patient or a treated patient, at the time of sampling of a specimen described later. Type of treatment are not particularly limited, for which therapeutic methods are exemplified by surgery, radiation therapy, chemotherapy, and immunotherapy. Only one type, or a combination of two or more types of therapeutic method for lung cancer is applicable.

The specimen is not particularly limited as long as it contains miRNA derived from lung cancer cells. The specimen is preferably a biological sample collected from the lung cancer patient, and is typically exemplified by tissue, cell, blood, lymph fluid, sputum, and bronchoalveolar lavage fluid. Among them, preferred is tissue, cell, and blood. The tissue preferably contains lung cancer tissue, or is the lung cancer tissue per se. The cell preferably contains lung cancer cell, or is the lung cancer cell per se. For example, lung cancer tissue or lung cancer cell collected from a site of occurrence of lung cancer, by surgery or biopsy, is usable as the specimen. Alternatively, a culture obtained by culturing lung cancer tissue or lung cancer cell collected from a lung cancer patient may be used as the specimen. Alternatively, plasma or serum prepared from blood by a usual method may be used as the specimen.

The specimen may be stored under conditions capable of stably maintaining RNA, up to measurement of miRNA described later. The specimen is typically cryopreserved at -20°C or below, and preferably at -80°C or below. The specimen, if being a tissue or cell collected from a subject, may be stored in a commercially available storage solution for cell or tissue. Such storage solution is exemplified by RNAlater (registered trademark) stabilization solution (Invitrogen). The specimen, if being the tissue collected from the subject, may be stored as a formalin-fixed paraffin-embedded (FFPE) sample. That is, the method of this embodiment may use the FFPE sample as the specimen.

The measurement sample may be prepared, typically by extracting RNA from a specimen of a lung cancer patient. The measurement sample may typically be an RNA solution that contains miRNA. RNA may be extracted from the specimen by any of known methods. RNA may be extracted from the specimen, if being a cell or tissue, typically as described below. First, the specimen is mixed with a solubilizing solution that contains guanidine thiocyanate and a surfactant. The obtained mixed solution is physically treated (stirring, homogenization, ultrasonic disintegration, etc.) to liberate RNA contained in the specimen into the mixed solution. Thus, RNA may be extracted from the specimen. The extracted RNA may be purified. For example, RNA may be purified by centrifuging the mixture that contains RNA, collecting a supernatant, followed by extraction from the supernatant with phenol/chloroform. The solubilizing solution used herein may be a commercially available reagent such as TRIzol (registered trademark) reagent, or ISOGEN II (Nippon Gene Co., Ltd.). Usable herein may be a commercially available reagent kit for extracting the miRNA-containing total RNA, such as miRNeasy (registered trademark) mini kit (QIAGEN), or High Pure miRNA Isolation kit (Roche).

RNA may be extracted from the specimen, if being an FFPE sample, typically as described below. First, xylene is applied to the FFPE sample to deparaffinize it. The deparaffinized sample is immersed in ethanol to be hydrophilized. The hydrophilized specimen is digested with a protease to release nucleic acid having been crosslinked with formalin, thereby extracting RNA from the tissue in the FFPE sample. Usable herein may be a commercially available reagent kit for extracting and purifying RNA from FFPE sample, such as miRNeasy (registered trademark) FFPE kit (QIAGEN).

The measurement sample may alternatively be prepared by reverse transcription of RNA extracted from a specimen of a lung cancer patient. For example, the measurement sample may be a solution that contains cDNA obtainable by reverse transcription of RNA that contains miRNA. Alternatively, the measurement sample may be prepared by in vitro transcription (IVT) amplification of cDNA derived from the specimen. For example, the measurement sample may be a solution that contains cRNA, obtainable by IVT amplification of cDNA synthesized from RNA that contains miRNA.

miR-21C is a variant of miR-21, having cytosine (C) added to the 3' end of the base sequence of miR-21. The base sequences of miR-21C and miR-21 are as follows.

miR-21C: 5'- UAGCUUAUCAGACUGAUGUUGAC -3' (SEQ ID NO: 1)
miR-21: 5'- UAGCUUAUCAGACUGAUGUUGA -3' (SEQ ID NO: 2)

The method for measuring miR-21C is not particularly limited, for which any of known methods is applicable. For example, the methods are exemplified by measurement method based on hybridization with a probe, measurement method based on nucleic acid amplification method, measurement method based on mass spectrometry, and measurement method based on sequencing. The measurement method based on hybridization with a probe is exemplified by microarray method, northern hybridization method, and RNase protection assay. The measurement method based on nucleic acid amplification is preferably quantitative PCR with use of, as a template, cDNA obtained by reverse transcription of miRNA, and is exemplified by quantitative RT-PCR method and quantitative RT-LAMP method. The quantitative RT-PCR method is exemplified by SYBR (trademark) Green method, and TaqMan (registered trademark) method. Since the miRNA is short, the method based on nucleic acid amplification may alternatively employ a stem-loop primer, or poly-(A) addition method. This embodiment may use a commercially available reagent kit for miRNA quantification, such as TaqMan (registered trademark) MicroRNA reverse transcription kit (Applied Biosystems), and TaqMan (registered trademark) MicroRNA assays (Applied Biosystems).

miR-21C and miR-21 are measured for calculation of the relative value of the miR-21C measurement value to the miR-21 measurement value. In this technical field, miR-21C and miR-21 in a measurement sample may be measured separately, typically by properly designing base sequences of a primer and/or a probe.

The expression level of miR-21C is a value that corresponds to the abundance of miR-21C in the measurement sample. This is a value which may be acquired typically by measuring miR-21C (hereinafter, also referred to as "miR-21C measurement value"). The miR-21C measurement value is exemplified by optically measured value, the number of reaction cycles (Ct value) or reaction time at which the optically measured value reached a predetermined reference value, and quantitative value of miR-21C. The optically measured value is exemplified by fluorescence intensity, turbidity, and absorbance. The quantitative value of miR-21C is exemplified by the number of copies, mass, and concentration. The predetermined reference value may be properly set, depending on the type of value that indicates the abundance or concentration of miR-21C. In an exemplary case with use of the quantitative RT-PCR method, amount of change in the fluorescence intensity, in a nucleic acid amplification reaction that occurs under logarithmic amplification, may be set with reference to a predetermined number of cycles as the reference value. Alternatively, the miR-21C measurement value may be standardized with use of measurement value of an endogenous control, obtained by measuring the endogenous control which serves as an internal standard. The endogenous control is exemplified by RNU48 small nuclear RNA (snRNA), RNU6B snRNA, and TBP (TATA box binding protein) mRNA. The expression level of miR-21C may be a relative value of the miR-21C measurement value to the miR-21 measurement value (hereinafter, also simply referred to as a "relative value"). The expression level of miR-21C conceptually covers a value that is calculated from the miR-21C measurement value or the relative value within the scope of common technical knowledge in the art. More specifically, the expression level is exemplified by power or power root of the aforementioned value, and a value obtainable by any of four arithmetic operations of the aforementioned value with a predetermined constant.

The expression level of miR-21C is preferably given by a relative value of the miR-21C measurement value to the miR-21 measurement value. This relative value may be calculated by dividing the N-th power of the miR-21C measurement value by the miR-21 measurement value, as given by equation (I) below. In equation (I), N represents a positive integer, which is preferably 1 or 2. Given N = 2, the relative value obtainable by equation (I) is also referred to as D-score.

(Relative value)=(miR-21C measurement value)$^N$/(miR-21 measurement value)          Equation (I)

If the miR-21C and miR-21 measurement values are given by the Ct values, the relative value of the miR-21C measurement value to the miR-21 measurement value used herein may be a value calculated by subtracting the Ct value of miR-21 from the Ct value of miR-21C to find a value ($\Delta$Ct), and then by substituting the value ($\Delta$Ct) into the equation (II) below. Alternatively, a value calculated by the comparative Ct method (also referred to as $\Delta\Delta$Ct method) may be used as the relative value. The value calculated by the comparative Ct method is a relative expression level of miR-21C, with reference to the expression level of miR-21. The comparative Ct method is known per se.

$$(\text{Relative value}) = 2^{-(\Delta Ct)} \text{ ... Equation (II)}$$

As described later in Examples, the overall survival rate of a patient group with high expression level of miR-21C was found to be significantly better than that of a patient group with low expression level of miR-21C. It was also found, as described later in Examples, that the recurrence-free survival rate of a patient group with high expression level of miR-21C was significantly higher than that of a patient group with low expression level of miR-21C. Thus, the expression level of miR-21C can be an index of the prognosis of lung cancer.

The prognosis of lung cancer is preferably determined by comparing the expression level of miR-21C with a predetermined threshold value. For example, a poor prognosis of lung cancer is suggested by the expression level of miR-21C equal to or above a predetermined threshold value. A good prognosis of lung cancer is suggested by the expression level of miR-21C below the predetermined threshold value.

The prognosis of lung cancer is preferably determined by comparing a relative value of the miR-21C measurement value to the miR-21 measurement value, with a predetermined threshold value that corresponds to the relative value. For example, a poor prognosis of lung cancer is suggested by the relative value equal to or above the predetermined threshold value that corresponds to the relative value. A good prognosis of lung cancer is suggested by the relative value below the predetermined threshold value that corresponds to the relative value.

Another embodiment of the method of the present invention relates to a method for assisting determination of prognosis of lung cancer, the method including the steps of: measuring an expression level of miR-21C; and determining prognosis of lung cancer in a lung cancer patient, from the expression level of miR-21C. The step of measuring the expression level of miR-21C is as described previously. The "expression level of miR-21C" is as described previously.

In the determination step, the prognosis of lung cancer in the lung cancer patient is preferably determined with reference to a result of comparison between the expression level of miR-21C and a predetermined threshold value. More specifically, a poor prognosis of lung cancer is determined if the expression level of miR-21C is equal to or above a predetermined threshold value. A good prognosis of lung cancer is determined if the expression level of miR-21C is below the predetermined threshold value.

The predetermined threshold value that corresponds to the expression level of miR-21C may be properly set, without special limitation. For example, specimens are collected from a plurality of lung cancer patients, and miR-21C and miR-21 in the measurement samples are measured. Survival of the patients and the recurrence of lung cancer are checked and recorded every lapse of a certain period (one month to 12 months, for example) from the day the specimen were collected. Such recording is continued typically for 2 to 15 years, and data of the expression level of miR-21C are classified into data regarding a patient group with good prognosis, and data of a patient group with poor prognosis. A value that can most accurately distinguish the two patient groups is then determined to be set as the threshold value. The threshold value may be set typically taking sensitivity, specificity, positive predictive value, and negative predictive value into consideration.

Another embodiment of the present invention relates to a method for treatment of lung cancer. The method for treatment includes the steps of: measuring an expression level of miR-21C in a measurement sample prepared from a specimen of a lung cancer patient having been treated for lung cancer; determining prognosis of lung cancer from the expression level of miR-21C; and providing adjuvant chemotherapy to the lung cancer patient whose prognosis of lung cancer was determined to be poor. Details of the measuring step and the determining step are same as those described previously in relation to the method of this embodiment.

Drugs usable for the adjuvant chemotherapy are known per se, and may be suitably determined depending on type and stage of lung cancer. The drugs are exemplified by tegafur-uracil, cisplatin, carboplatin, vinorelbine, docetaxel, paclitaxel, pemetrexed, vindesine, and an EGFR tyrosine kinase inhibitor. The drugs may be used singly or in combination of two or more kinds thereof.

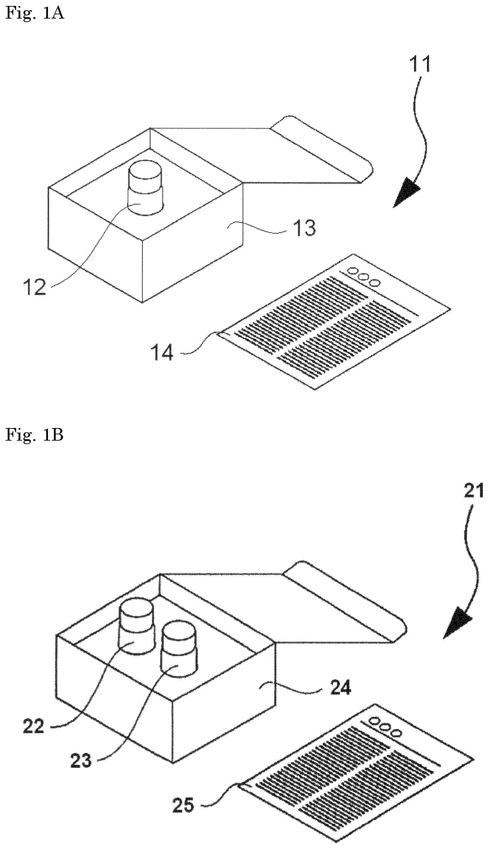

Another embodiment of the present invention relates to a reagent kit (hereinafter, referred to as "reagent kit of this embodiment"). The reagent kit contains a primer set that specifically hybridizes to cDNA derived from miR-21C. The primer set includes a forward primer and a reverse primer that specifically hybridize to cDNA derived from miR-21C. An exemplary reagent kit is illustrated in Fig. 1A. In Fig. 1A, reference numeral 11 denotes a reagent kit, reference numeral 12 denotes a first vial that contains a primer set capable of specifically binding to cDNA derived from miR-21C, reference numeral 13 denotes a packaging box, and reference numeral 14 denotes a package insert. The package insert may typically contain descriptions on sequences of the individual primers, method of use such as PCR conditions, and instructions for storage.

The reagent kit of this embodiment may further include a primer for reverse transcription (hereinafter, also referred to as "RT primer"). For use in a two-step, or sequential process consisting of synthesis of cDNA from miRNA, and amplification of cDNA, the RT primer may be a random primer or a primer that specifically hybridizes to miR-21C. For use in a one-step, or simultaneous process consisting of synthesis of cDNA from miRNA, and amplification of cDNA, the RT primer used herein is a primer that specifically hybridizes to miR-21C.

The reagent kit of this embodiment may further contain a primer set that specifically hybridizes to cDNA derived from miR-21. The primer set includes a forward primer and a reverse primer that specifically hybridize to cDNA derived from miR-21. An exemplary reagent kit is illustrated in Fig. 1B. In Fig. 1B, reference numeral 21 denotes a reagent kit, reference numeral 22 denotes a first vial that contains a primer set capable of specifically hybridizing to cDNA derived from miR-21C, reference numeral 23 denotes a second vial that contains a primer set capable of specifically hybridizing to cDNA derived from miR-21, reference numeral 24 denotes a packaging box, and reference numeral 25 denotes a package insert.

If the reagent kit of this embodiment contains, as the RT primer, a primer that specifically hybridizes to miR-21C, the reagent kit may further contain a primer that specifically hybridizes to miR-21.

Each of the aforementioned primers may be properly designed corresponding to the individual base sequences of miR-21C and miR-21. Since miR-21C and miR-21 have short chain lengths, the RT primer and/or the primer set may be a stem-loop primer. The RT primer and/or the primer set may also be bound to a solid phase such as plate or particle.

The reagent kit of this embodiment may further contain a probe that specifically hybridizes to cDNA derived from miR-21C. The reagent kit of this embodiment may further contain a probe that specifically hybridizes to cDNA derived from miR-21. Each of these probes is preferably modified at the 5'end with a fluorescent substance, and at the 3' end with a quencher substance. These probes are also preferably designed to hybridize to a region different from the region in which the aforementioned primer set hybridizes, in cDNA derived from miR-21C or miR-21. In the cDNA amplification method with use of such probes, an amplification product is detectable quantitatively, by detecting fluorescence emitted from the probes.

In this specification, "capable of specifically hybridizing" means that the primer or the probe can hybridize to a target nucleic acid molecule, under a stringent condition. In this embodiment, the target nucleic acid molecule is miR-21C, cDNA derived from miR-21C, miR-21, or cDNA derived from miR-21. The "stringent condition" means a condition under which a target nucleic acid molecule can be hybridized with the primer or the probe, to a degree that is detectably larger than a degree a nucleic acid molecule other than the target nucleic acid molecule can be hybridized (more than at least twice the background, for example). The stringent condition is usually sequence-dependent, and varies in various environments. The stringent condition is usually set approximately 5°C lower than a thermal melting point (Tm) of a particular sequence at a specified ionic strength and pH. The Tm is a temperature at which 50% of a probe complementary to the base sequence of the target nucleic acid molecule can hybridize in equilibrium, at a specified ionic strength, pH, and nucleic acid composition. Such condition may only be a condition used for a known method for hybridization between polynucleotides.

The reagent kit of the present embodiment may further contain a DNA polymerase and/or reverse transcriptase. The DNA polymerase is exemplified by Taq polymerase, Pfu polymerase, and Tth polymerase. The reverse transcriptase is exemplified by AMV reverse transcriptase and MMLV reverse transcriptase. The reagent kit of this embodiment may further contain buffer, dNTPs (dATP, dTTP, dGTP and dCTP) or the like.

The aforementioned primer set is also understood to be used for manufacturing the reagent kit of this embodiment. That is, another embodiment of the present invention relates to use of a primer set that specifically hybridizes to cDNA derived from miR-21C, intended for manufacturing a reagent kit used in a method for assisting determination of prognosis of lung cancer. Yet another embodiment relates to use of a primer set that specifically hybridizes to cDNA derived from miR-21C, and a primer set that specifically hybridizes to cDNA derived from miR-21, intended for manufacturing a reagent kit used in a method for assisting determination of prognosis of lung cancer. Details of the method for assisting determination of prognosis of lung cancer, the reagent kit, and the primer set are as described previously.

The present invention will be further detailed below, referring to Examples, to which the present invention is by no means limited.

EXAMPLES

Example 1: Determination of Prognosis of Lung Cancer Based on miR-21C Measurement Value

1. Specimen

Lung cancer tissues were collected from a plurality of lung cancer patients with different lung cancer classifications and stages. Written informed consent was obtained from each patient. A part of tumor was collected from each of the patients during surgery, and individually cryopreserved in liquid nitrogen, or stored in RNAlater (registered trademark) Stabilization Solution (Invitrogen).

2. Quantitative RT-PCR

Total RNA was extracted from each of the collected tissues with use of TRIzol (registered trademark) reagent (Invitrogen). The obtained total RNA was quantified with use of Qubit (registered trademark) RNA HS assay kit (Invitrogen). RNA extraction and quantification were conducted as instructed by the package inserts of the reagent and the kit. With use of TaqMan (registered trademark) MicroRNA Reverse Transcription Kit (Applied Biosystems), 10 ng of total RNA was reversely transcribed. With use of the thus obtained cDNA as a template, quantitative PCR was conducted with use of TaqMan (registered trademark) Fast Advanced Mastermix (Applied Biosystems) and TaqMan (registered trademark) MicroRNA assays (Applied Biosystems), to measure miR-21C and miR-21. Also RNU48 snRNA, RNU6B snRNA, or TBP mRNA, used as an internal control, were measured in the same manner. All PCR was conducted in triplicate with use of Applied Biosystems ViiA (registered trademark) 7 quantitative PCR system. Amplification conditions include a cycle consisting of 95°C for 1 second and 60°C for 20 seconds, repeated 40 times.

3. Analysis

Statistical analysis was conducted with use of medical statistical analysis software Prism (GraphPad). Patients with the miR-21C measurement values equal to or above a predetermined threshold value were classified into a "miR-21C exp. high" group, while those with the miR-21C measurement values below the predetermined threshold value were classified into a "miR-21C exp. low" group. Similarly, patients with the miR-21 measurement values equal to or above a predetermined threshold value were classified into a "miR-21 exp. high" group, while those with the miR-21 measurement values below the predetermined threshold value were classified into a "miR-21 exp. low" group. Differences in the measurement values were assessed by the Student's t-test. Survival curves in terms of overall survival (OS) of the patients in the individual groups were prepared. Also survival curves in terms of OS of the patients with stage-I and -II lung cancers in the individual groups were prepared. Differences in OS were assessed by the log-rank test. A $p$ value of less than 0.05 (p < 0.05) was considered as statistically significant.

4. Results

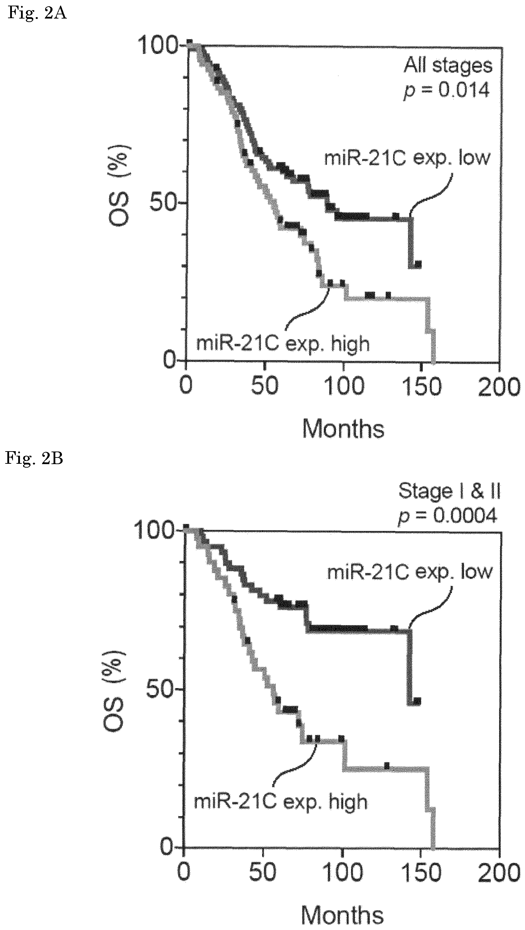

The survival curves thus prepared are illustrated in Figs. 2A to 2D. As illustrated in Fig. 2A, the miR-21C exp. low group tends to demonstrate OS significantly higher than that of the miR-21C exp. high group. That is, it was suggested that the higher the expression level of miR-21C, the poorer the prognosis, and the lower the expression level, the better the prognosis. As illustrated in Fig. 2B, early (stage-I and -II) lung cancers demonstrated distinct difference in OS, between the miR-21C exp. high group and the miR-21C exp. low group. The result suggests that the early-stage lung cancer may be more accurately determined for the prognosis, from the miR-21C measurement values.

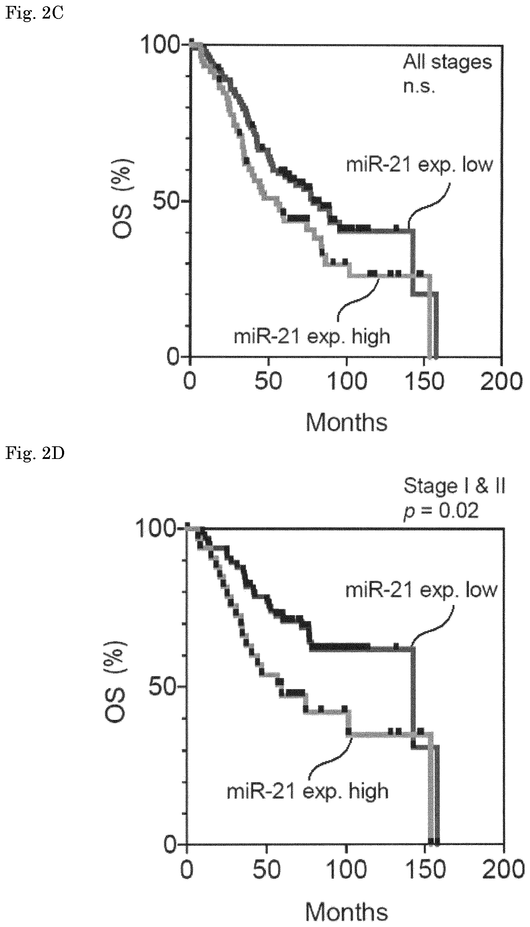

In contrast as illustrated in Figs. 2C, no significant differences in OS was observed between the miR-21 exp. high group and the miR-21 exp. low group which were classified by the miR-21 measurement values. As illustrated in Figs. 2D, a significant difference in OS was observed between the miR-21 exp. high group and the miR-21 exp. low group, for early-stage lung cancer. Comparison with the results illustrated in Fig. 2B, however, taught that higher accuracy of prediction of the prognosis was given by the determination based on the miR-21C measurement values.

Example 2: Determination of Recurrence Risk of Lung Adenocarcinoma Based on miR-21C measurement value

1. Specimen

Lung adenocarcinoma tissues were collected from a plurality of lung adenocarcinoma patients in different stages. Written informed consent was obtained from each patient. A part of tumor was collected from each of the patients during surgery, and cryopreserved in liquid nitrogen, or stored in RNAlater (registered trademark) Stabilization Solution (Invitrogen).

2. Quantitative RT-PCR and Analysis

Reverse transcription and quantitative RT-PCR were conducted with use of total RNA extracted from the collected tissues, in the same manner as in Example 1. Data of the acquired measurement values of miR-21C and miR-21 were statistically analyzed with use of Prism (GraphPad), in the same manner as in Example 1. The lung adenocarcinoma patients were classified by the measurement values of miR-21C and miR-21, similarly to as in Example 1. Survival curves in terms of recurrence-free survival rate (RFS) of the patients in the individual groups were prepared. Also survival curves in terms of RFS of the patients with stage-I and -II lung adenocarcinoma in the individual groups were prepared. Differences in RFS were assessed by the log-rank test. A p value of less than 0.05 (p < 0.05) was considered as statistically significant.

3. Results

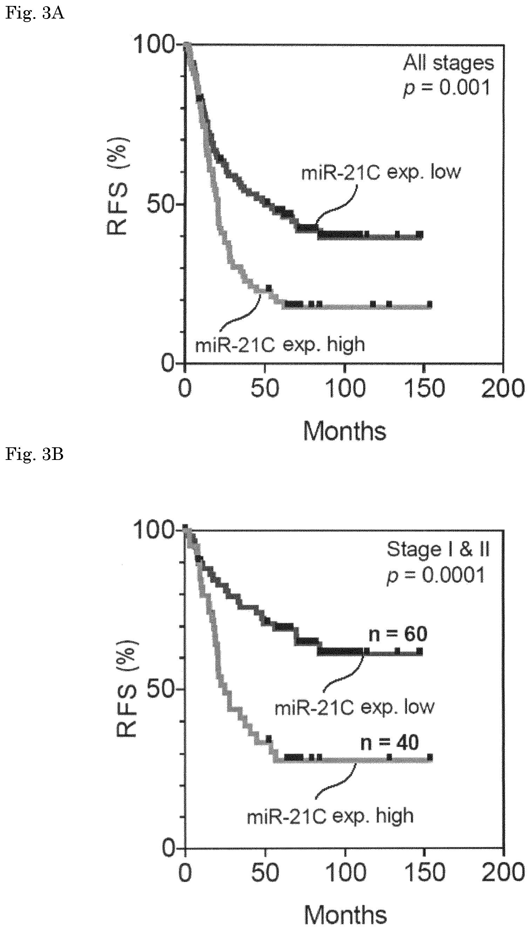

The survival curves thus prepared are illustrated in Figs. 3A to 3D. As illustrated in Fig. 3A, the miR-21C exp. low group tends to demonstrate RFS significantly higher than that of the miR-21C exp. high group. That is, it was suggested that the higher the miR-21C measurement value, the higher the recurrence risk, and the lower the measurement value, the lower the recurrence risk. As illustrated in Fig. 3B, early (stage-I and -II) lung adenocarcinoma demonstrated distinct difference in RFS, between the miR-21C exp. high group and the miR-21C exp. low group. The result suggests that the early lung adenocarcinoma may be more accurately determined for the recurrence risk, from the miR-21C measurement values.

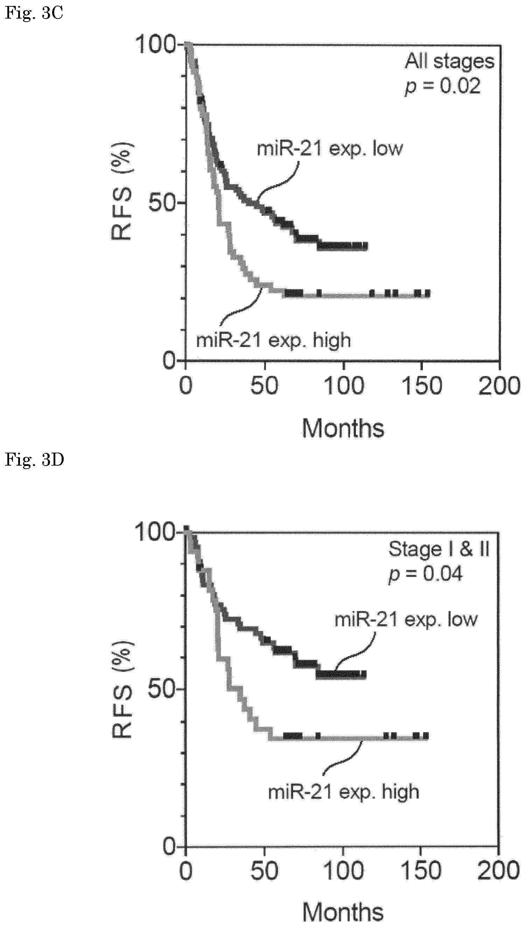

As illustrated in Figs. 3C and 3D, significant differences in RFS were observed between the miR-21 exp. high group and the miR-21 exp. low group, for early lung adenocarcinoma in all stages. Comparison with the results illustrated in Figs. 3A and 3B, however, taught that higher accuracy of prediction of the recurrence risk was given by the determination based on the miR-21C measurement values.

Example 3: Determination of Recurrence Risk of Lung Cancer Based on Relative Value of miR-21C Measurement Value to miR-21 Measurement Value

1. Specimens and Analysis

With use of the measurement data of the lung cancer tissue specimen of Example 1, a relative value was calculated by the comparative Ct method, from difference in Ct values with the same threshold value. Detailed procedures are as follows. First, Ct values of miR-21C, miR-21 and an internal control were calculated at threshold = 1.5. Next, the Ct value of the internal control was subtracted from each of the Ct values of miR-21C and miR-21, to standardize the Ct values of miR-21C and miR-21. The standardized Ct value of miR-21 was then subtracted from the standardized Ct value of miR-21C to obtain a difference ($\Delta$Ct), which was then substituted into the equation below to calculate the relative value.

$$(\text{Relative value}) = 2^{\wedge} - (\Delta\text{Ct})$$

Patients with the relative values equal to or above a predetermined threshold value were classified into a "miR-21C/miR-21 high" group, while those with the relative values below the predetermined threshold value were classified into a "miR-21C/miR-21 low" group. Differences in the relative values were assessed by the Student's t-test. Survival curves in terms of RFS of the patients in the individual groups were prepared. Also survival curves in terms of RFS of the patients with stage-I and -II lung cancers in the individual groups were prepared. Differences in RFS were assessed by the log-rank test. A p value of smaller than 0.05 ($p < 0.05$) was regarded as statistically significant.

2. Results

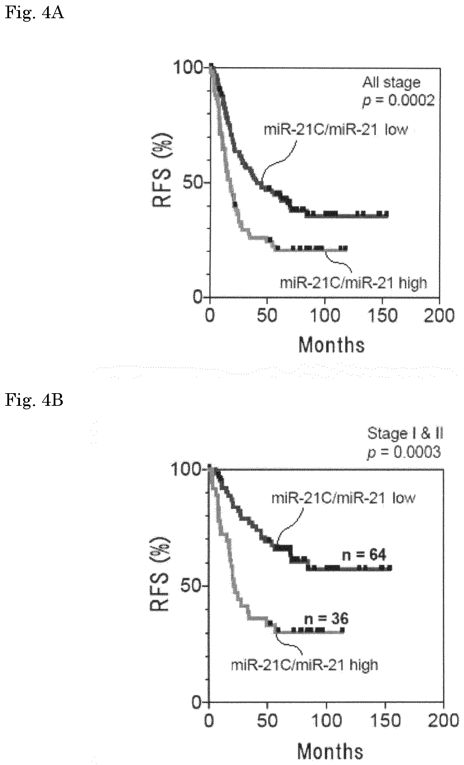

The survival curves thus prepared are illustrated in Figs. 4A and 4B. As illustrated in Fig. 4A, the miR-21C/miR-21 low group tends to demonstrate RFS significantly higher than that of the miR-21C/miR-21 high group. That is, it was suggested that the higher the relative value, the higher the recurrence risk, and the lower the relative value, the lower the recurrence risk. As illustrated in Fig. 4B, early (stage-I and -II) lung cancer demonstrated distinct difference in RFS, between the miR-21C/miR-21 high group and the miR-21C/miR-21 low group. The determination of recurrence risk based on the relative value was thus suggested to be more suitable for early-stage lung cancer.

Example 4: Determination of Recurrence Risk of Lung Adenocarcinoma Based on D-Score

1. Specimens and Analysis

Measurement data of the lung adenocarcinoma tissue specimen of Example 2 was used. More specifically, the D-score was calculated by the following equation, with use of miR-21C and miR-21 measurement values.

$$(\text{D-score}) = (\text{miR-21C measurement value})^2/(\text{miR-21 measurement value})$$

Patients with the D-scores equal to or above a predetermined threshold value were classified into a "High" group, while those with the D-scores below the predetermined threshold value were classified into "Low" group. Differences in the D-scores were assessed by the Student's t-test. Survival curves in terms of RFS of the patients in the individual groups were prepared. Also survival curves in terms of RFS of the patients with stage-I and -II lung adenocarcinoma in the individual groups were prepared. Also survival curves in terms of RFS of the patients with well-differentiated lung adenocarcinoma in all stages in the individual groups were prepared. Differences in RFS were assessed by the log-rank test. A p value of less than 0.05 ($p < 0.05$) was considered as statistically significant.

2. Results

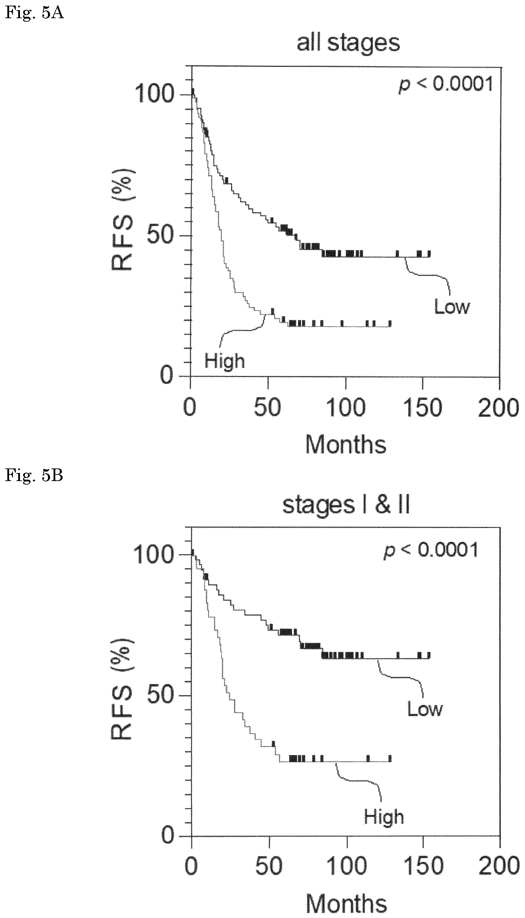

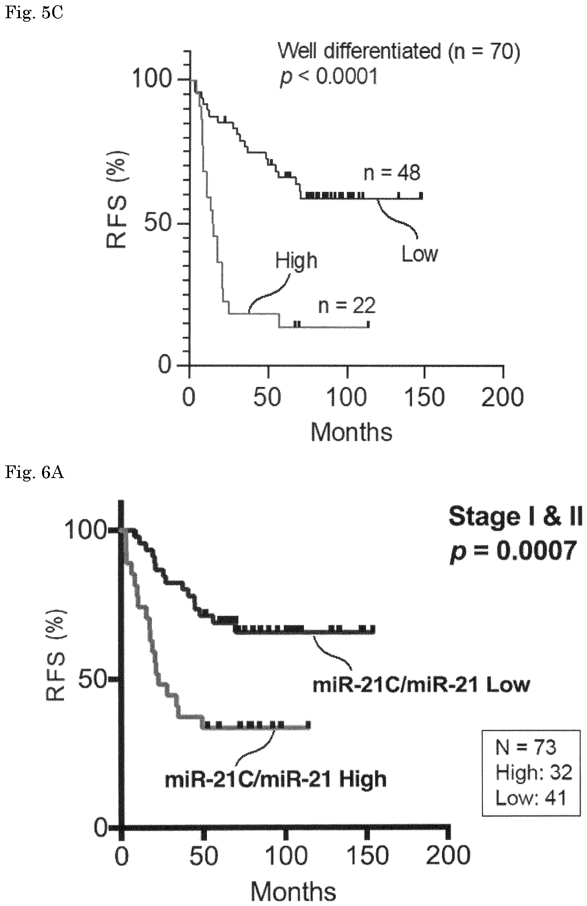

The survival curves thus prepared are illustrated in Figs. 5A to 5C. As illustrated in Fig. 5A, the Low group tends to demonstrate RFS significantly higher than that of the High group. That is, it was suggested that the higher the D-score, the higher the recurrence risk, and the lower the D-score, the lower the recurrence risk. As illustrated in Fig. 5B, similar results were obtained also for early stage (stage-I and -II) lung cancers. Significant differences in RFS with a p value of smaller than 0.0001 were found between the High group and the Low group, both for the all stages, and for the early stages. That is, it was suggested that the recurrence risk may be determined very accurately, with use of the D-score.
As illustrated in Fig. 5C, the Low group tends to demonstrate RFS significantly higher than that of the High group, also for the well-differentiated lung adenocarcinoma. The result demonstrated that the recurrence risk of highly-differentiated lung adenocarcinoma may be clearly stratified with the D-score.

Example 5: Comparison with p53 Gene Mutation, an Existing Prognostic Predictor

1. Specimen

A lung cancer tissue was collected from each of the lung cancer patients different from those in Example 1, and cryopreserved in liquid nitrogen, or stored in RNAlater (registered trademark) Stabilization Solution (Invitrogen).

2. Quantitative RT-PCR and Analysis

Reverse transcription and quantitative RT-PCR were conducted with use of total RNA extracted from the collected tissues, to measure miR-21C and miR-21, in the same manner as in Example 1. Also p53 gene mutation was detected by sequence analysis. Statistical analysis was conducted with use of medical statistical analysis software Prism (GraphPad).

Similarly to as in Example 3, patients with the relative values of miR-21C measurement values to the miR-21 measurement value equal to or above a predetermined threshold value were classified into a "miR-21C/miR-21 high" group, while those with the relative values below the predetermined threshold value were classified into a "miR-21C/miR-21 low" group. Also patients without p53 gene mutation were classified into a "p53 WT" group, while patients having p53 gene mutation were classified into a "p53 MUT" group. Survival curves in terms of RFS of the patients with stage-I and -II lung cancers in the individual groups were prepared. Differences in RFS were assessed by the log-rank test. Ap value of less than 0.05 ($p < 0.05$) was considered as statistically significant.

3. Results

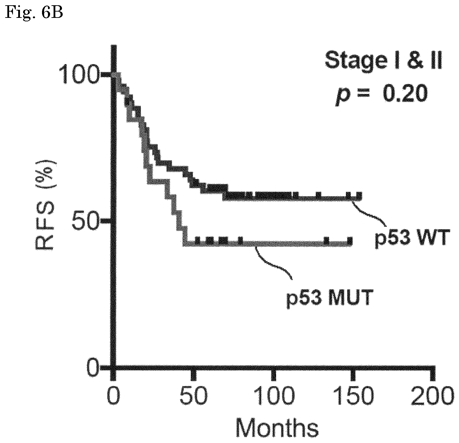

The survival curves thus prepared are illustrated in Figs. 6A and 6B. As illustrated in Fig. 6A, the miR-21C/miR-21 low group tends to demonstrate RFS significantly higher than that of the miR-21C/miR-21 high group. In contrast as illustrated in Fig. 6B, no significant difference in RFS was observed between the p53 WT group and the p53 MUT classified based on the presence or absence of mutations in p53 gene. It was thus demonstrated that the determination of the recurrence risk based on the relative value of the miR-21C measurement value to the miR-21 measurement value was more accurate than the determination based on the p53 gene mutation.

DESCRIPTION OF REFERENCE SIGNS

11, 21: reagent kit
12, 22: first vial
23: second vial
13, 24: packaging box
14, 25: package insert

**Claims**

1. A method for assisting determination of prognosis of lung cancer, the method comprising measuring miR-21C in a measurement sample prepared from a specimen of a lung cancer patient, an expression level of miR-21C acquired by the measurement serving as an index of the prognosis of lung cancer.

2. The method according to claim 1, wherein

   a poor prognosis of lung cancer is suggested by an expression level of miR-21C equal to or above a predetermined threshold value, and
   a good prognosis of lung cancer is suggested by an expression level of miR-21C below the predetermined threshold value.

3. The method according to claim 1, further comprising measuring miR-21 in the measurement sample.

4. The method according to claim 3, wherein the expression level of miR-21C is given by a relative value of a miR-21C measurement value to a miR-21 measurement value.

5. The method according to claim 4, wherein

   the relative value is a value calculated by an equation below:

   (Relative value)=(miR-21C measurement value)$^{N}$/(miR-21 measurement value)

   where, N represents a positive integer.

6. The method according to claim 5, wherein N represents 1 or 2.

7. The method according to claim 5 or 6, wherein

   a poor prognosis of lung cancer is suggested by a relative value equal to or above a predetermined threshold value that corresponds to the relative value, and
   a good prognosis of lung cancer is suggested by a relative value below the predetermined threshold value that corresponds to the relative value.

8. A method for assisting determination of prognosis of lung cancer, the method comprising the steps of:

   measuring miR-21C in a measurement sample prepared from a specimen of a lung cancer patient; and
   determining the prognosis of lung cancer, with reference to an expression level of miR-21C acquired by the measurement.

9. The method according to claim 8, wherein

   in the determining step,
   the prognosis of lung cancer is determined to be poor, if the expression level of miR-21C is equal to or above a predetermined threshold value, and
   the prognosis of lung cancer is determined to be good, if the expression level of miR-21C is below the predetermined threshold value.

10. The method according to claim 8, wherein, in the measuring step, miR-21 in the measurement sample is further measured.

11. The method according to claim 10, wherein the expression level of miR-21C is given by a relative value of a miR-21C measurement value to a miR-21 measurement value.

12. The method according to claim 11, wherein

    the relative value is a value calculated by an equation below:

    (Relative value)=(miR-21C measurement value)$^{N}$/(miR-21 measurement value)

    where, N represents a positive integer.

13. The method according to claim 12, wherein N represents 1 or 2.

14. The method according to claim 12 or 13, wherein

    in the determining step,
    the prognosis of lung cancer is determined to be poor, if the relative value is equal to or above a predetermined threshold value that corresponds to the relative value, and
    the prognosis of lung cancer is determined to be good, if the relative value is below the predetermined threshold value that corresponds to the relative value.

15. The method according to any one of claims 1 to 14, wherein the lung cancer is early-stage lung cancer.

16. The method according to claim 15, wherein the early-stage lung cancer is stage-I or -II lung cancer.

17. The method according to any one of claims 1 to 16, wherein the lung cancer is adenocarcinoma.

18. The method according to any one of claims 1 to 17, wherein the specimen contains a lung cancer tissue.

19. A reagent kit for use in the method according to any one of claims 1 to 18, the reagent kit comprising a primer set that specifically hybridizes to a cDNA derived from miR-21C.

20. The reagent kit according to claim 19, further comprising a primer set that specifically hybridizes to a cDNA derived from miR-21.

Fig. 1A

Fig. 1B

Fig. 2A

Fig. 2B

Fig. 2C

Fig. 2D

Fig. 3A

Fig. 3B

Fig. 3C

Fig. 3D

Fig. 4A

Fig. 4B

Fig. 5A

all stages

Fig. 5B

stages I & II

Fig. 5C

Fig. 6A

Fig. 6B

## INTERNATIONAL SEARCH REPORT

| | International application No. |
|---|---|
| | **PCT/JP2022/033651** |

### A. CLASSIFICATION OF SUBJECT MATTER

*C12Q 1/6851*(2018.01)i; *C12N 15/113*(2010.01)i; *C12Q 1/6886*(2018.01)i
FI:  C12Q1/6851 Z ZNA; C12N15/113 Z; C12Q1/6886 Z

According to International Patent Classification (IPC) or to both national classification and IPC

### B. FIELDS SEARCHED

Minimum documentation searched (classification system followed by classification symbols)

C12Q1/00-3/00; C12N15/00-15/90

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); Japio-GPG/FX

### C. DOCUMENTS CONSIDERED TO BE RELEVANT

| Category* | Citation of document, with indication, where appropriate, of the relevant passages | Relevant to claim No. |
|---|---|---|
| Y | LEIKIN, J. D. et al. Analysis of miR-21 isomiRs in lung cancer. Cancer Res. 2015, vol. 75, no. 15_Supplement, LB-295 <br> particularly, abstract | 1-20 |
| Y | BOELE, J. et al. PAPD5-mediated 3' adenylation and subsequent degradation of miR-21 is disrupted in proliferative disease. Proc. Natl. Acad. Sci. U.S.A. 2014, vol. 111, no. 31, pp. 11467-11472 <br> particularly, p. 11468, left column, fifth paragraph, fig. 1 | 1-20 |
| A | JP 2009-531018 A (THE OHIO STATE UNIVERSITY RESEARCH FOUND.) 03 September 2009 (2009-09-03) <br> see entire text | 1-20 |
| A | US 2012/0130648 A1 (SKRZYPSKI, M.) 24 May 2012 (2012-05-24) <br> see entire text | 1-20 |
| A | WO 2020/022361 A1 (FUKUSHIMA MEDICAL UNIV.) 30 January 2020 (2020-01-30) <br> see entire text | 1-20 |

☑ Further documents are listed in the continuation of Box C.   ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **19 October 2022** | **01 November 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
|---|
| **PCT/JP2022/033651** |

**C.    DOCUMENTS CONSIDERED TO BE RELEVANT**

| Category* | Citation of document, with indication, where appropriate, of the relevant passages | Relevant to claim No. |
|---|---|---|
| A | WO 2013/191242 A1 (JAPANESE FOUND. FOR CANCER RESEARCH) 27 December 2013 (2013-12-27)<br>　　see entire text | 1-20 |
| A | VOLINIA, S. et al. A microRNA expression signature of human solid tumors defines cancer gene targets. Proc. Natl. Acad. Sci. U.S.A. 2006, vol. 103, no. 7, pp. 2257-2261<br>　　see entire text | 1-20 |

Form PCT/ISA/210 (second sheet) (January 2015)

## INTERNATIONAL SEARCH REPORT

International application No.

**PCT/JP2022/033651**

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    "The form of Annex C/ST.25 text file" above shall read as "the form of ST.26.".

Form PCT/ISA/210 (continuation of first sheet) (January 2015)

**INTERNATIONAL SEARCH REPORT**
**Information on patent family members**

International application No.

**PCT/JP2022/033651**

| Patent document cited in search report | | | Publication date (day/month/year) | Patent family member(s) | | | Publication date (day/month/year) |
|---|---|---|---|---|---|---|---|
| JP | 2009-531018 | A | 03 September 2009 | WO | 2007/081720 | A2 | |
| | | | | see entire text | | | |
| | | | | US | 2008/0306017 | A1 | |
| | | | | EP | 1978986 | A2 | |
| US | 2012/0130648 | A1 | 24 May 2012 | WO | 2011/014083 | A2 | |
| | | | | EP | 2459748 | A2 | |
| WO | 2020/022361 | A1 | 30 January 2020 | (Family: none) | | | |
| WO | 2013/191242 | A1 | 27 December 2013 | JP | 2014-3925 | A | |

Form PCT/ISA/210 (patent family annex) (January 2015)

**EP 4 428 248 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8361710 B